# EUROPEAN PATENT APPLICATION

(11) **EP 4 260 867 A1**
(43) Date of publication of application: **18.10.2023**
(21) Application number: 20964997.9
(22) Date of filing: 09.12.2020
(51) Int. Cl.: A61K 38/17, A61P 37/02

(54) **PEPTIDE COMPOSITION FOR IMMUNOTHERAPY**

(71) Applicant: Instituto Politecnico Nacional, Zacatenco Ciudad De México, 07738 (MX)
(72) Inventor: PÉREZ TAPIA, Sonia Mayra, Del. Miguel Hidalgo CDMX, 11340 (MX); VALLEJO CASTILLO, Luis Alberto, Del. Miguel Hidalgo CDMX, 11340 (MX)
(74) Representative: De Clercq & Partners
(86) International application number: PCT/IB2020/061715
(87) International publication number: WO 2022/123298

(57) **Abstract**

It is an object of the present invention to provide a peptide composition with immunomodulatory effects for immunotherapy that does not exhibit variability in peptide content according to its cellular origin and manufacturing method. To this end, a peptide composition with immunomodulatory effects has been invented which comprises the complete human Ubiquitin (Ub) monomer, and a variant of the human Ubiquitin monomer which lacks two terminal glycines (Ub(-GG)). The peptide composition for immunotherapy can further comprise at least a fragment of the following human proteins: Ankyrin-1, Protein 4.1, Hemoglobin Alpha Subunit, Hemoglobin Beta Subunit, Hemoglobin Delta Subunit, Complement Protein C3, Calpastatin, Alpha-Synuclein, Fibrinogen Alpha Chain, Cytoplasmic Actin 1, Thymosin Beta-4, Thymosin Beta-10, or Zixin.

## Description

### FIELD OF THE INVENTION

The present invention is related to immunotherapies, and more particularly it is related to a peptide composition with immunomodulatory effects.

### BACKGROUND OF THE INVENTION

Immunotherapy involves the stimulation, enhancement, suppression, or desensitization of the immune response to treat conditions with an inflammatory component. Among the immunotherapeutics, the human dialyzable leukocyte extracts (hDLE) stand out, which are made of a mixture of low molecular weight peptides with immunomodulatory properties. From their first description, made in 1949 by Henry Sherwood Lawrence, dialyzable leukocyte extracts have been used as therapeutic aids to treat diseases related to immune system deregulation. However, the biological effects of these cell extracts have been controversial due to the lack of information about the identity and variability of the content that they present between them, mainly due to the lack of reliable information about their source of origin and the manufacturing method thereof, which makes it impossible to elucidate their mechanism of action

A dialyzable leukocytes extract has been described comprising a complex mixture of low molecular weight (<10 kDa) peptides obtained by dialysis/filtration of human leukocyte lysates, and its active ingredient is a peptides complex mixture reproducible between batches according to the method described in the patent document WO2013089550. This product, derived from human blood, is the active ingredient of oral or injectable pharmaceutical forms and is used in the treatment of diseases with an inflammatory component (Medina-Rivero, E., et al., Batch-to-batch reproducibility of Transferon. J Pharm Biomed Anal, 2014. 88: p. 289-94; and Estrada-Parra, S., et al., Comparative study of transfer factor and acyclovir in the treatment of herpes zoster. Int. J. Immunopharmacol, 1998. 20(10): p. 521-535.).

The preclinical and clinical information that has been generated indicates that the aforementioned dialyzable leukocytes extract modulates the production of proinflammatory cytokines (Salinas-Jazmin, N., et al., Herpes murine model as a biological assay to test dialyzable leukocyte extracts activity. J Immunol Res, 2015. 146305: p. 1 - 9; and Santacruz-Valdez, C., et al., Dialyzable leukocyte extracts (transfer factor) as adjuvant therapy for fungal keratitis. Am J case rep, 2010. 11: p. 97 - 101.). Furthermore, it improves the resolution of infectious diseases by increasing the number of IFN-gamma-producing cells in ocular fungal keratitis (Santacruz-Valdez, C., et al., Dialyzable leukocyte extracts (transfer factor) as adjuvant therapy for fungal keratitis. Am J case rep, 2010. 11: p. 97 - 101.) and improves the survival of pediatric patients with sepsis by decreasing the serum concentration of C-reactive protein (CRP), increases the total number of lymphocytes, and decreases the total number of neutrophils (Castrejón-Vázquez, MI., et al., Dialyzable Leukocyte Extract (Transferon™) Administration in Sepsis: Experience from a Single Referral Pediatric Intensive Care Unit. Biomed Res Int, 2019. 8980506: 2 - 10.). Furthermore, this dialyzable leukocyte extract increases serum IFN-gamma levels and favors the clinical course in patients with herpes zoster infection when administered subcutaneously (Estrada-Parra, S., et al., Comparative study of transfer factor and acyclovir in the treatment of herpes zoster. Int. J. Immunopharmacol, 1998. 20(10): p. 521-535). Various models have been developed to elucidate the mechanisms by which this orally dialyzable leukocytes extract causes its therapeutic effect. As regards the *in vitro* models, this dialyzable leukocyte extract added to the culture medium increases the expression of CD80/CD86 and IL-6 levels in THP-1 cells stimulated with LPS (Jiménez-Uribe, A., et al. CD80 Expression Correlates with IL-6 Production in THP-1-Like Macrophages Costimulated with LPS and Dialyzable Leukocyte Extract (Transferon®). J Immunol Res, 2019. 2198508: p. 1 - 9), while it induces the differentiation of IFN-gamma producing CD56+CD16+CD11c+ NK cells from CD34+ progenitor cells obtained from human umbilical cord (Ramírez-Ramírez, D., et al. Early Differentiation of Human CD11c(+)NK Cells with γδ T Cell Activation Properties Is Promoted by Dialyzable Leukocyte Extracts. J Immunol Res, 2016. 4097642: p 1 - 12). Similarly, in tumor models, this orally administered dialyzable leukocytes extract (1-25 µg/kg) reduces tumor growth and metastasis in a murine model of prostate cancer (Hernández-Esquivel, M., et al. The dialyzable leukocyte extract Transferon™ inhibits tumor growth and brain metastasis in a murine model of prostate cancer. Biomed Pharmacother, 2018. 101 p: 938 - 944), while in the murine model of Herpes Simplex Virus type 1 (HSV-1) infection, it decreases the blood levels of TNF-alpha and IL-6, and increases IFN-gamma levels and the percentage of survival when administered oropharyngealy (Salinas-Jazmin, N., et al., Herpes murine model as a biological assay to test dialyzable leukocyte extracts activity. J Immunol Res, 2015. 146305: p. 1 - 9). However, the information available from these models is not sufficient to explain how a mixture of peptides can modulate the immune system when administered orally or parenterally. Therefore, it is essential to identify the most abundant peptide components of the referred dialyzable leukocytes extract, to understand the bases of the immunomodulatory properties of this hemoderivative.

Until now, only one report on the identification of peptide sequences in dialyzable extracts derived from mammalian cells (mice and bovines) is known, dating from the year 2000 (Kirkpatrick, C.H., Transfer factors: identification of conserved sequences in transfer factor molecules. Mol Med, 2000. 6(4): p. 332-41). In this work, the sequence of conserved non-human peptides was obtained by Edman degradation sequencing. However, these peptide sequences have not been found in any public information database, despite the fact that the human, murine and bovine proteomes are currently known, mammals from which the peptides analyzed in said reference were obtained. Although Edman degradation is still a widely used sequencing technique, its effectiveness is limited to samples with high purity peptides. Therefore, this technique is not suitable for the analysis of complex mixtures such as the dialyzable leukocytes extract described because these products are composed of a myriad of peptides with a low concentration. For the identification and quantification of a large number of peptides in complex samples, different methods based on mass spectroscopy (peptidomic studies) are used.

On the other hand, patent document US 2007/0207162 describes Ubiquitin compositions or fragments of said protein to suppress the immune system or treat sepsis in a mammal. Nevertheless, said document is limited to describing the use of ubiquitin or a fragment of said protein to suppress the immune system when it is exacerbated, but not to stimulate it when it is depressed. Furthermore, patent document US 2007/0207162 does not describe Ubiquitin compositions in combination with fragments of said protein, and even less with fragments of other human proteins for the integral regulation (stimulatory and suppressor) of the immune system.

As a consequence of the foregoing, efforts have been made to eliminate the drawbacks of the hDLE currently used for immunotherapy, developing a peptide composition with immunomodulatory effects that does not present variability in the peptide content according to its cellular origin and manufacturing method.

### OBJECTIVES OF THE INVENTION

Taking into account the shortcomings of the prior art, it is an object of the present invention to provide a peptide composition with immunomodulatory effects that does not exhibit variability in peptide content according to its cellular origin and manufacturing method and that is useful for the comprehensive regulation of the immune system.

These and other objects are achieved by a peptide composition with immunomodulatory effects according to the present invention.

### BRIEF DESCRIPTION OF THE INVENTION

To this end, a peptide composition with immunomodulatory effects has been invented which comprises the complete human Ubiquitin (Ub) monomer, and a variant of the human Ubiquitin monomer that lacks two terminal glycines (Ub(-GG)).

In a preferred embodiment of the present invention, the peptide composition with immunomodulatory effects additionally comprises at least one peptide fragment of the following human proteins: Ankyrin-1, Protein 4.1, Hemoglobin Alpha Subunit, Hemoglobin Beta Subunit, Hemoglobin Delta Subunit, Complement Protein C3, Calpastatin, Alpha-Synuclein, Fibrinogen Alpha Chain, Cytoplasmic Actin 1, Thymosin Beta-4, Thymosin Beta-10, or Zixin.

### BRIEF DESCRIPTION OF THE FIGURES

Aspects of novelty which are considered characteristic of the present invention will be established with particularity in the appended claims. However, some embodiments, features and some objects and advantages thereof, will be better understood in the detailed description, when read in connection with the attached drawings, in which:
Figure 1 shows the percentage contribution of protein fragments to the total mass spectrum of a dialyzable leukocytes extract.
Figures 2A-2B shows the analysis of the percentage and coverage of the proteins that contribute peptides to a dialyzable leukocytes extract.
Figure 3 shows the analysis of the abundance of peptides in a dialyzable leukocytes extract, which is reproducible in 10 batches.
Figures 4A-4C shows the identification of the complete human Ubiquitin (Ub) monomer in a dialyzable leukocytes extract using a commercial standard monomeric Ub as a reference.
Figures 5A-5C shows verification of the presence of the complete human Ubiquitin (Ub) monomer and a variant of the human Ubiquitin monomer lacking two terminal glycines (Ub(-GG)) in a dialyzable leukocytes extract using a Ub standard as reference.
Figure 6 shows an MS analysis of reduced samples of a dialyzable extract of leukocytes where the TIC and m/z signals are attributable to Ub and Ub(-GG) and not to molecular aggregates.
Figure 7 shows that the presence of Ub and Ub(-GG) is consistent across batches of the dialyzable leukocytes extract.
Figures 8A-8B shows the identification of Ub and Ub(-GG) using polyclonal antibodies.
Figure 9 shows the structural comparison of Ub and Ub(-GG) and the quantification of total Ub in a dialyzable leukocytes extract.
Figure 10 shows the protective effect of a dialyzable leukocytes extract and Ub in the murine model of HSV-1 infection.

### DETAILED DESCRIPTION OF THE INVENTION

A peptide composition has been found to be useful for use in immunotherapy for the stimulation, enhancement, suppression or desensitization of the immune response to treat conditions with an inflammatory component.

Thus, in one aspect of the invention, a peptide composition with immunomodulatory effects is described which comprises the complete human Ubiquitin (Ub) monomer and a variant of the human Ubiquitin monomer that lacks two terminal glycines (Ub(-GG)).

The complete Ub monomer preferably has a molecular mass of about 8.56 kDa. Likewise, the Ub(-GG) monomer variant preferably has a molecular mass of about 8.45 kDa. The Ub monomer is a natural serum globular protein of small size (76 amino acids) that lacks post-translational modifications and has high thermal, structural, and proteolytic stability.

The complete Ub monomer is preferably selected from UBC_HUMAN (UniProtKB Accession Number P0CG48) or from UBB_HUMAN (UniProtKB Accession Number P0CG47). More preferably the complete Ub monomer is selected from the amino acid sequence of SEQ ID NO: 1.

Likewise, the Ub(-GG) monomer variant is preferably selected from the fragment J3QRK5_HUMAN (UniProtKB Accession Number UPI000268AF41) or from the ribosomal variants RS27A_HUMAN (UniProtKB Accession Number P62979) and RL40_HUMAN (UniProtKB Accession Number P62987). More preferably, the Ub(-GG) monomer variant is selected from the amino acid sequence of SEQ ID NO: 2.

Preferably, the peptide composition with immunomodulatory effects comprises from 0.168 to 0.268% (w/w) of the complete Ub monomer and of the Ub(-GG) monomer variant. The remainder of the peptide composition is made up of minor peptide fragments. More preferably, the average content of the Ub(-GG) monomer variant in the peptide composition with immunomodulatory effects is in a 2:1 ratio to the average content of the complete Ub monomer.

In a preferred embodiment of the present invention, the peptide composition with immunomodulatory effects additionally comprises at least one peptide fragment of the following human proteins: Ankyrin-1, Protein 4.1, Hemoglobin Alpha Subunit, Hemoglobin Beta Subunit, Hemoglobin Delta Subunit, Complement Protein C3, Calpastatin, Alpha-Synuclein, Fibrinogen Alpha Chain, Cytoplasmic Actin 1, Thymosin Beta-4, Thymosin Beta-10, or Zixin.

For the purposes of the present invention, the terms "peptide fragment" or "fragment" mean a continuous segment of a protein, which has a molecular mass of less than 10 kDa.

Preferably, the peptide composition with immunomodulatory effects comprises from 0.644 to 0.924% (w/w) of at least one Ankyrin-1 fragment. Likewise, preferably, the peptide composition with immunomodulatory effects comprises from 0.397 to 0.637% (w/w) of at least one Protein 4.1 fragment. Likewise, preferably, the peptide composition with immunomodulatory effects comprises from 0.351 to 0.571% (w/w) of at least one Hemoglobin Alpha Subunit fragment. Likewise, preferably, the peptide composition with immunomodulatory effects comprises from 0.367 to 0.547% (w/w) of at least one fragment of the Hemoglobin Beta Subunit and/or of the Hemoglobin Delta Subunit. Likewise, preferably, the peptide composition with immunomodulatory effects comprises from 0.303 to 0.583% (w/w) of at least one Complement C3 protein fragment. Likewise, preferably, the peptide composition with immunomodulatory effects comprises from 0.319 to 0.519% (w/w) of at least one Calpastatin fragment. Likewise, preferably, the peptide composition with immunomodulatory effects comprises from 0.198 to 0.298% (w/w) of at least one Alpha-Synuclein fragment. Likewise, preferably, the peptide composition with immunomodulatory effects comprises from 0.151 to 0.311% (w/w) of at least one Fibrinogen Alpha Chain fragment. Likewise, preferably, the peptide composition with immunomodulatory effects comprises from 0.163 to 0.283% (w/w) of at least one Cytoplasmic Actin 1 fragment. Likewise, preferably, the peptide composition with immunomodulatory effects comprises from 0.12 to 0.2% (w/w) of at least one Thymosin Beta-4 and/or Thymosin Beta-10 fragment. Likewise, preferably, the peptide composition with immunomodulatory effects comprises from 0.108 to 0.208% (w/w) of at least one Zixin fragment.

In a preferred embodiment of the present invention, each peptide fragment is selected from the human Ankyrin-1 protein, which in turn is preferably selected from ANK1_HUMAN (UniProtKB Accession Number P16157) or Q6PK32_HUMAN (UniProtKB Accession Number Q6PK32). More preferably, each peptide fragment corresponds to a fragment of the amino acid sequence of SEQ ID NO: 3 or SEQ ID NO: 4.

In another preferred embodiment of the present invention, each peptide fragment is selected from the human Protein 4.1 protein preferably selected from 41_HUMAN (UniProtKB Accession Number P11171). More preferably, each peptide fragment corresponds to a fragment of the amino acid sequence of SEQ ID NO: 5.

In another preferred embodiment of the present invention, each peptide fragment is selected from the human Hemoglobin Alpha Subunit protein preferably selected from HBA_HUMAN (UniProtKB Accession Number P69905) or from G3V1N2_HUMAN (Uniprot Accession Number G3V1N2). More preferably, each peptide fragment corresponds to a fragment of the amino acid sequence of SEQ ID NO: 6 or SEQ ID NO: 7.

In another preferred embodiment of the present invention, each peptide fragment is selected from the human Hemoglobin Beta Subunit protein preferably selected from HBB_HUMAN (UniProtKB Accession Number P68871). More preferably, each peptide fragment corresponds to a fragment of the amino acid sequence of SEQ ID NO: 8.

In another preferred embodiment of the present invention, each peptide fragment is selected from the human Hemoglobin Delta Subunit protein preferably selected from E9PEW8_HUMAN (UniProtKB Accession Number E9PEW8). More preferably, each peptide fragment corresponds to a fragment of the amino acid sequence of SEQ ID NO: 9.

In another preferred embodiment of the present invention, each peptide fragment is selected from the human Complement C3 protein preferably selected from CO3_HUMAN (UniProtKB Accession Number P01024). More preferably, each peptide fragment corresponds to a fragment of the amino acid sequence of SEQ ID NO: 10.

In another preferred embodiment of the present invention, each peptide fragment is selected from the human Calpastatin protein, preferably selected from ICAL_HUMAN (UniProtKB Accession Number P20810) or from A0A0A0MR45_HUMAN (UniProtKB Accession Number A0A0A0MR45), or from D6RC54_HUMAN (UniProtKB Accession Number access D6RC54 from UniProtKB). More preferably, each peptide fragment corresponds to a fragment of the amino acid sequence of SEQ ID NO: 11, SEQ ID NO: 12 or SEQ ID NO: 13.

In another preferred embodiment of the present invention, each peptide fragment is selected from the human Alpha-Synuclein protein preferably selected from SYUA_HUMAN (UniProtKB Accession Number P37840) or H6UYS7_HUMAN (UniProtKB Accession Number H6UYS7). More preferably, each peptide fragment corresponds to a fragment of the amino acid sequence of SEQ ID NO: 14 or SEQ ID NO: 15.

In another preferred embodiment of the present invention, each peptide fragment is selected from the human Fibrinogen Alpha Chain protein preferably selected from FIBA_HUMAN (UniProtKB Accession Number P02671). More preferably, each peptide fragment corresponds to a fragment of the amino acid sequence of SEQ ID NO: 16.

In another preferred embodiment of the present invention, each peptide fragment is selected from the human Cytoplasmic Actin 1 protein preferably is selected from ACTB_HUMAN (UniProtKB Accession Number P60709), from I3L3R2_HUMAN (UniProtKB Accession Number I3L3R2), or from POTEE_HUMAN (UniProtKB Accession Number Q6S8J3). More preferably, each peptide fragment corresponds to a fragment of the amino acid sequence of SEQ ID NO: 17, SEQ ID NO: 18 or SEQ ID NO: 19.

In another preferred embodiment of the present invention, each peptide fragment is selected from the human Thymosin Beta-4 protein preferably selected from TYB4_HUMAN (UniProtKB Accession Number P62328). More preferably, each peptide fragment corresponds to a fragment of the amino acid sequence of SEQ ID NO: 20.

In another preferred embodiment of the present invention, each peptide fragment is selected from the human Thymosin Beta-10 protein preferably selected from TYB10_HUMAN (UniProtKB Accession Number P63313). More preferably, each peptide fragment corresponds to a fragment of the amino acid sequence of SEQ ID NO: 21.

In another preferred embodiment of the present invention, each peptide fragment is selected from the human Zixin protein preferably selected from ZYX_HUMAN (UniProtKB Accession Number Q15942). More preferably, each peptide fragment corresponds to a fragment of the amino acid sequence of SEQ ID NO: 22.

The peptide composition with immunomodulatory effects can be administered in combination with other pharmaceutical and/or immunogenic and/or immunostimulatory and/or immunotherapeutic agents and can be combined with a physiologically or pharmaceutically acceptable vehicle. For example, the peptide composition with immunomodulatory effects can be administered together with cytokines, adjuvants, or antibodies.

Immunologically effective amounts and method of administration of the peptide composition with immunomodulatory effects may vary depending on the individual, the condition to be treated, and other factors apparent to one skilled in the art. Factors to consider include but are not limited to: the route of administration, the number of doses, whether the peptide composition with immunomodulatory effects will be administered together with other pharmaceutical and/or immunogenic and/or immunostimulatory agents or a physiologically or pharmaceutically acceptable vehicle thereof. Such factors are known in the art, and it is within the knowledge of those skilled in the art to make such determinations. A suitable dosage range is one that provides the desired modulation of the immune response. Useful dosage ranges of the peptide composition with immunomodulatory effects can be, for example, from about any of the following: 0.1 to 100 µg/kg, 0.1 to 50 µg/kg, 0.1 to 25 µg/kg, 0.1 to 10 µg/kg, 1 to 500 µg/kg, 100 to 400 µg/kg, 200 to 300 µg/kg, 1 to 100 µg/kg, 100 to 200 µg/kg, 300 to 400 µg/kg, 400 to 500 µg/kg. Alternatively, the doses can be approximately any of the following: 0.1 µg, 0.25 µg, 0.5 µg, 1.0 µg, 2.0 µg, 5.0 µg, 10 µg, 25 µg, 50 µg, 75 µg, 100 µg. Therefore, dose ranges may be those with a lower limit of any of the following: 0.1 µg, 0.25 µg, 0.5 µg and 1.0 µg; and with an upper limit of about any of the following: 25 µg, 50 µg and 100 µg. The absolute amount administered to each patient depends on pharmacological properties such as bioavailability, clearance rate, and route of administration.

The effective amount and method of administration of the peptide composition with immunomodulatory effects may vary according to the individual patient and the stage of the disease and other factors apparent to one skilled in the art. The route(s) of administration useful in a particular application are apparent to one skilled in the art. Routes of administration include, but are not limited to, topical, dermal, transdermal, transmucosal, epidermal, parenteral, gastrointestinal, oral, oropharyngeal, nasopharyngeal, and pulmonary, including transbronchial and transalveolar. A suitable dosage range is one that provides sufficient peptide composition with immunomodulatory effects to achieve a preventive or therapeutic effect. The absolute amount administered to each patient depends on pharmacological properties such as bioavailability, clearance rate, and route of administration.

The present invention will be better understood from the following Examples, which are presented solely for illustrative purposes to allow a full comprehension of the preferred embodiments of the present invention, without implying that there are no other non-illustrated embodiments that may be put into practice based on the detailed description above.

### EXAMPLES

### Example 1. Obtaining a dialyzable leukocyte extract and de novo sequencing by mass spectrometry.

An assay was performed to identify the peptide components of a dialyzable leukocytes extract through mass spectrometry techniques.

All batches of the dialyzable leukocytes extract were manufactured by Pharma-FT Laboratory (Mexico City, Mexico) using a previously described standardized method (Medina-Rivero, E., et al., Physicochemical Characteristics of Transferon Batches. Biomed Res Int, 2016. 2016: p. 7935181.). Briefly, "leuko-platelet and erythrocyte concentrates" were received from certified human blood banks and the cell contents were broken by applying freeze-thaw cycles. The cell lysate was dialyzed through a 12 kDa membrane and the permeate was filtered through a 10 kDa cartridge. The obtained solution was diluted to 0.4 mg/mL using water for injection. The transferred batches underwent quality control analysis, including sterility, identity, pH, endotoxin content, relative density, total protein content, identity, and potency. All batches of the dialyzable leukocytes extract were kept at -18°C until use.

The dialyzable leukocytes extract was sequenced *de novo* to identify the proteins that provide its peptide content. Ten batches of the dialyzable leukocytes extract (14G18, 14G19, 15A03, 15A04, 15C10, 15D11, 15D12, 15E14, 15F17 and 15G18) were sequenced using standardized methods. In this sense, the samples of the dialyzable leukocytes extract were lyophilized, and 2 mg of the dialyzable leukocytes extract were purified using the ProteoExtract^{®} Protein Precipitation Kit according to the manufacturer's instructions. Samples were reconstituted in about 100 µL of 6.0 M urea, reduced with 5 mM 1-4 dithiothreitol at 37°C for 30 min, and alkylated with 15 mM iodoacetamide at room temperature for 10 min. The iodoacetamide was quenched by adding an excess of the reducing agent. Trypsin/Lys-C was added in a ratio of 1:25 (enzyme:peptide) and incubated at 37°C for 4 h. Then, 550 µL of 50 mM ammonium bicarbonate buffer solution was added and digestion continued overnight. Samples were desalted using Macro Spin Columns^{®} according to the manufacturer's instructions. The samples were recovered in a solution of water: acetonitrile + formic acid (20%: 80% + 0.5%).

Samples were analyzed by tandem mass spectrometry coupled to liquid chromatography (MS/MS) (LC-MS/MS) on a Thermo Scientific Q Exactive + Orbitrap mass spectrometer coupled with a Proxeonnanospray Proxeon Easy-nLC II HPLC source (Thermo Scientific^{®}). Peptides were separated using a 75 µm x 150 mm Magic C18 200Å 3U reverse phase column and a 120 min gradient with a 300 nL/min flow. Data was obtained from 350-1600 m/z where the top ions were subjected to high energy collisional dissociation (HCD), and a normalized collision energy of 27% was used for fragmentation. Tandem mass spectra were extracted by Proteome Discoverer^{®} version 1.2 charge state deconvolution and deisotopes were not performed. All MS/MS samples were analyzed using X! Tandem (The GMP group; https://www.thegpm.org/), was created to search the NCBI database of human refseq and non-human common contaminants and an equal number of reverse sequences assuming trypsin digestion. The search yielded a fragment ion and parent ion mass tolerance of 20 ppm and 10 ppm, respectively. Scaffold v4.7.5 (Proteome Software Inc.) was used for data validation. Peptide identification was accepted if they met a threshold of 97% using the Scaffold LFDR algorithm, and protein identification was accepted if they contained at least two peptides at this threshold. Proteins sharing significant peptide evidence were grouped into groups. Scaffolding was also used to identify the most abundant peptides from the dialyzable leukocytes extract, which was based on signal intensity and probability of identification (>95%).

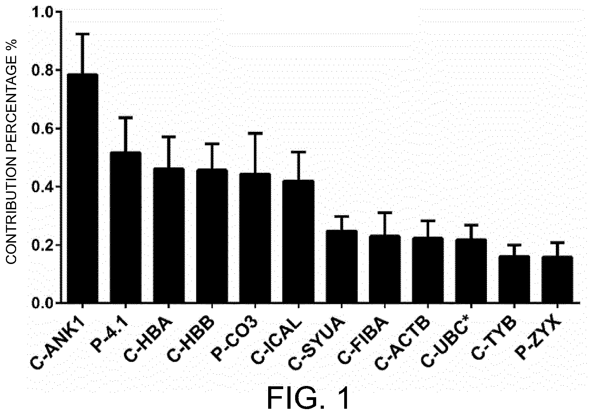

In this analysis, each sample of the dialyzable leukocytes extract was found to be composed of about 20,000 peptides derived from 593 human proteins. In this sense, the protein clusters were ordered from highest to lowest spectrometric intensity, and those that i) appeared consistently among the ten analyzed batches (reproducibility), ii) were identified with a probability greater than 95%, and iii) whose spectrometry intensity was above the signal/noise ratio, which was defined as the point where keratin sequences were detected, a common environmental contaminant in this type of analysis, were selected. It was determined that the most abundant peptides from the dialyzable leukocytes extract, according to the intensity of their mass spectrum, come from 22 human proteins, 18 of them grouped (Table 1). Overall, these peptides represent 4.319% (SD <1.03) of the total peptide content of the dialyzable leukocytes extract, with ankyrin (ANK-1) peptides contributing the most (Figure 1).

**Table 1**

| **UniProtKB Access** | **Percentage of total spectrum (%)** | **Standard deviation (SD)** |
|---|---|---|
| **1) ANK1_HUMAN** | 0.784 | 0.14 |
| **2) Q6PK32_HUMAN** | | |
| **3) 41_HUMAN** | 0.517 | 0.12 |
| **4) HBA_HUMAN** | 0.461 | 0.11 |
| **5) G3V1N2_HUMAN** | | |
| **6) HBB_HUMAN** | 0.457 | 0.09 |
| **7) E9PEW8_HUMAN** | | |
| **8) CO3_HUMAN** | 0.443 | 0.14 |
| **9) ICAL_HUMAN** | 0.419 | 0.10 |
| **10) AOAOAOMR45 _ HUMAN** | | |
| **11) D6RC54_HUMAN** | | |
| **12) SYUA_HUMAN** | 0.248 | 0.05 |
| **13) H6UYS7_HUMAN** | | |
| **14) FIBA_HUMAN** | 0.231 | 0.08 |
| **15) ACTB_HUMAN** | 0.223 | 0.06 |
| **16) I3L3R2_HUMAN** | | |
| **17) POTEE_HUMAN** | | |
| **18) UBC_HUMAN** | 0.218 | 0.05 |
| **19) J3QRK5_HUMAN** | | |
| **20) TYB4_HUMAN** | 0.160 | 0.04 |
| **21) TYB10_HUMAN** | | |
| **22) ZYX_HUMAN** | 0.158 | 0.05 |
| **Global values** | **4.319** | **1.03** |

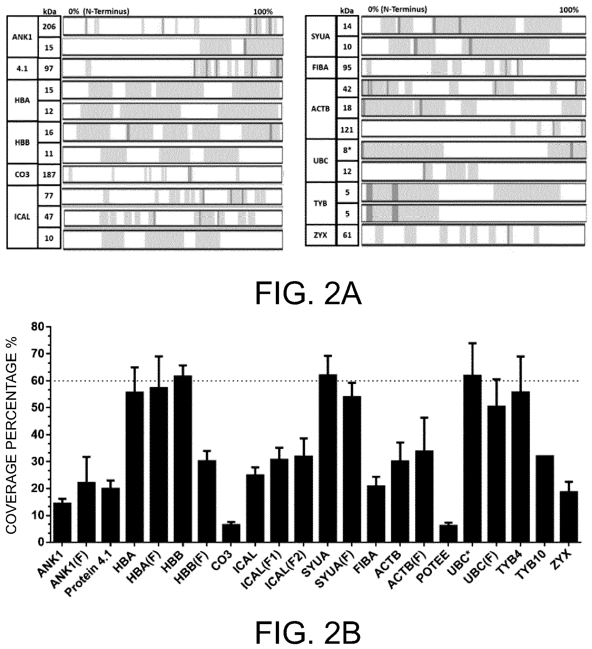

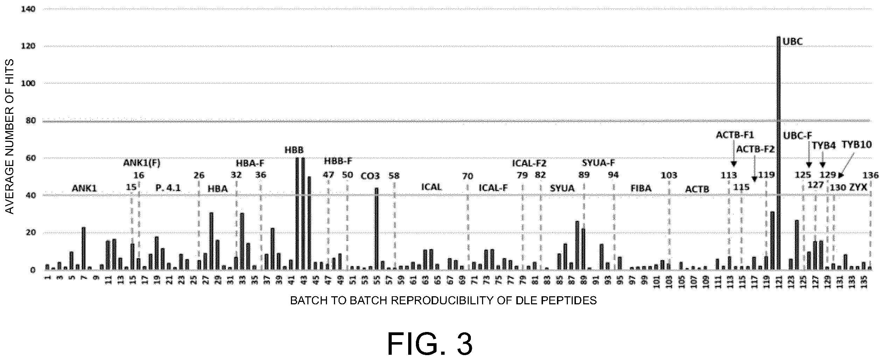

The contribution of each protein to the mass spectrum of a dialyzable leukocytes extract depends on: i) the number of peptides provided to the mixture and ii) the spectral intensity of each peptide. Therefore, the relative abundance per protein in the dialyzable leukocytes extract was qualitatively calculated based on the percentage coverage and the average number of hits of its peptides. The coverage map is seen in Figure 2A, while the coverage percentage is seen in Figure 2B. In addition, the mean number of hits per peptide was obtained as an indicator of relative abundance. In this regard, peptides that appeared in at least eight of the 10 sequenced batches were selected. 136 relevant peptides were identified, and their mean number of hits were grouped into a high (>80 hits) or intermediate (40-80) region of hits (Figure 3).

### Example 2. Intact mass analysis, identification of complete Ub monomer and Ub(-GG) monomer variant, and quantification of total Ub.

Intact mass analysis was performed to identify peptides from the dialyzable leukocytes extract with relevant biological activity.

The intact mass of the peptides from the dialyzable leukocytes extract was determined by mass spectrometry (MS) using the complete sequence of the proteins identified in the *de novo* sequencing of Example 1. Eight batches of the dialyzable leukocytes extract (16E16, 16E15, 16F17, 16F18, 16J27, 16J28, 16J29, 16H15 and 16H26) were lyophilized and reconstituted in 2 mg/mL MS water. Furthermore, a sample from batch 18A01 was reduced and acetylated as previously described. A 10 µL volume of samples of the dialyzable leukocytes extract and recombinant human Ub (1 µg/mL) was injected into a Vion^{®} ESI-IMS-Q-ToF coupled to an Acquity^{®} UPLC Class I system and separated with a CSH C18 of 1.7 µm (2.1 mm x 150 mm) for Ub identification and quantification and a BioSuite^{®} 3.0 µm C18 (2.1 x 150 mm) column for verification of Ub identity at 60°C. Samples were eluted at 0.2 mL/min using water + formic acid (0.1%) (Phase A) and acetonitrile + formic acid (0.1%) (Phase B) as follows: 100% from 0 to 10 min Phase A, and a 100 to 50% gradient from 10 to 85 min Phase A. IMS-QTof was operated in positive polarity and MSE/mode sensitivity from 50 to 2000 m/z. The collision energies were 5.00 eV (low), 10.00 eV (high), and 35.00 eV (end of high collision energy ramp). Electrospray ionization (ESI) parameters were set at: 150°C source temperature, 450°C desolvation temperature, 0 L/h cone gas, 1000 L/h desolvation gas, and 1000 L/h capillary voltage of 2.75kV. A 50 pg/µL leucine enkephalin solution (Waters, 556.2766 m/z) was infused during MS analysis at 5 µL/min for mass correction. Data was acquired and processed using Vion^{®} software in which the full sequence of proteins identified in *de novo* sequencing was loaded, and all possible peptides derived from specific LysC/trypsin digestion were searched with a tolerance of 25 ppm; no chemical or post-translational modifications were considered.

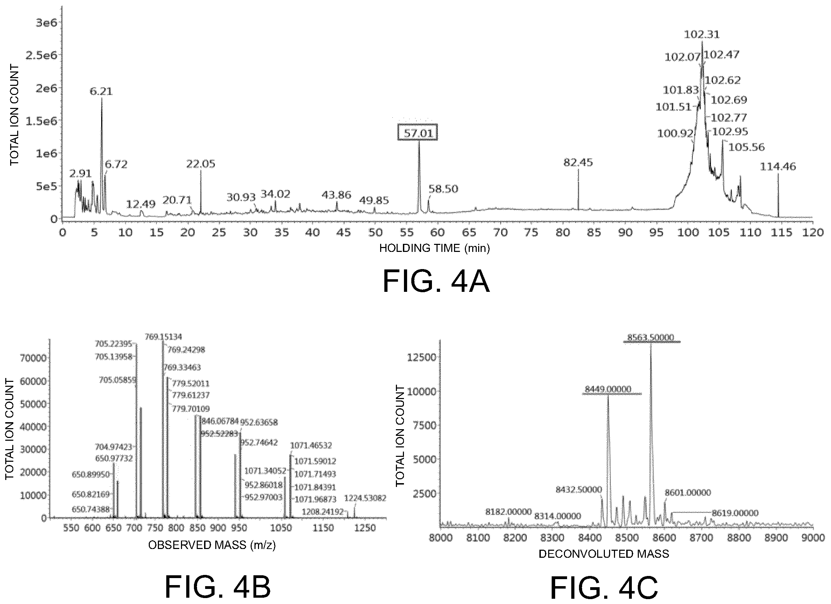

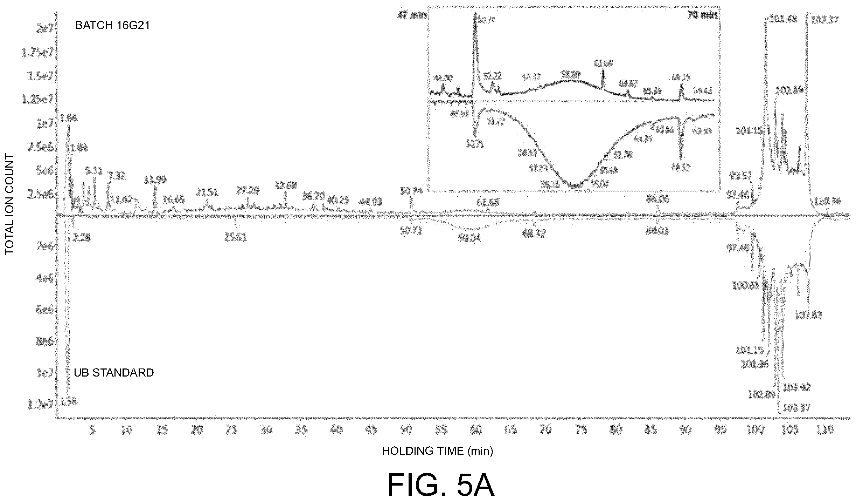

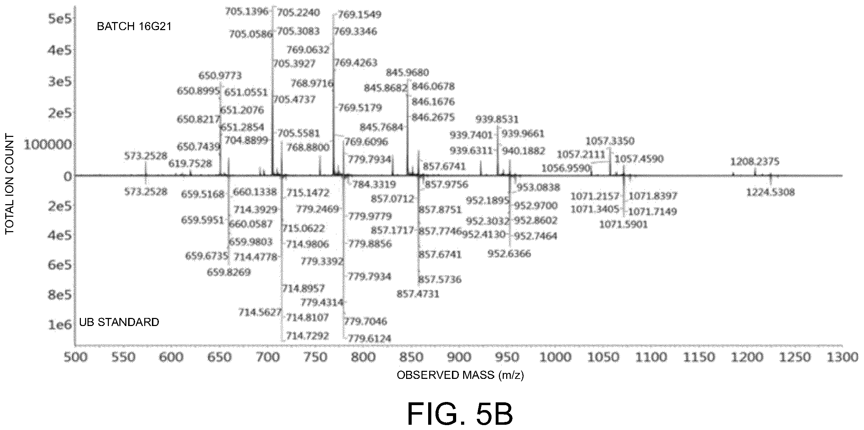

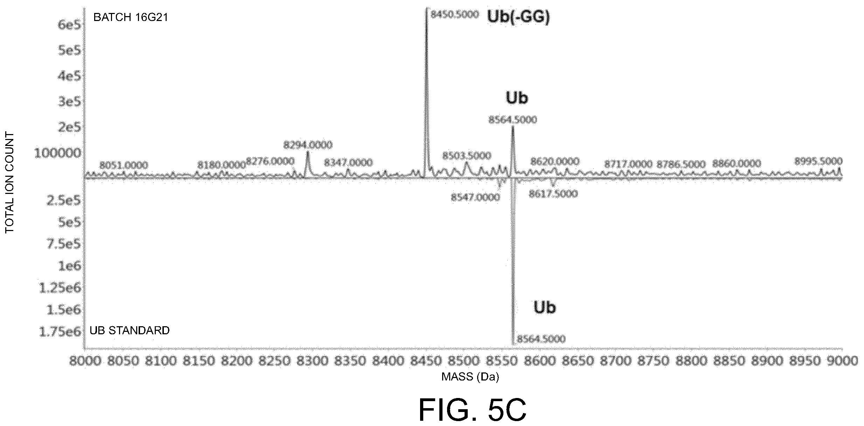

To identify peptides from the dialyzable leukocyte extract with a relevant biological activity, the most abundant component was determined, the highest Total Ion Count (TIC) signal was observed around 57 min (Figure 4A) using a 7µm CSH C18 column. Using the MaxEnt1 algorithm, the 57 min signal was combined, and the obtained m/z and its deconvoluted mass (Figure 4B and 4C, respectively) were very similar to the theoretical mass of the complete Ub monomer (8564.84 Da). A second peptide signal with a mass similar to the complete Ub monomer (8449.0 kDa) was observed in the same chromatographic peak. The presence of complete Ub monomer in the dialyzable leukocytes extract was verified by analyzing a pattern of the complete Ub monomer and the dialyzable leukocytes extract side by side using a 3.0 µ C18 BioSuite^{®} column; both samples exhibited the same TIC profile at the complete Ub monomer elution time, i.e., 50 min - 69 min (Figure 5A). The combination of the TIC profile of the dialyzable leukocytes extract from 50 to 69 min evidenced two sets of signals, as previously observed using the CSH C18 column of small particles, one of them was similar to the standard of the complete Ub monomer (Figure 5B). After the deconvolution using a MaxEnt1 algorithm, these sets of m/z signals from the dialyzable leukocytes extract were assigned to the complete Ub monomer (8564.50 Da) according to the intact mass of the standard, and the second set, the most intense, to a human Ubiquitin monomer variant lacking two terminal glycines (Ub(-GG)) (8450.5 kDa) according to the sequence predicted by the UNIPROT software and the theoretical mass calculation of Ub(-GG) using the ExPASy tool (8450.74 Da) (Figure 5C).

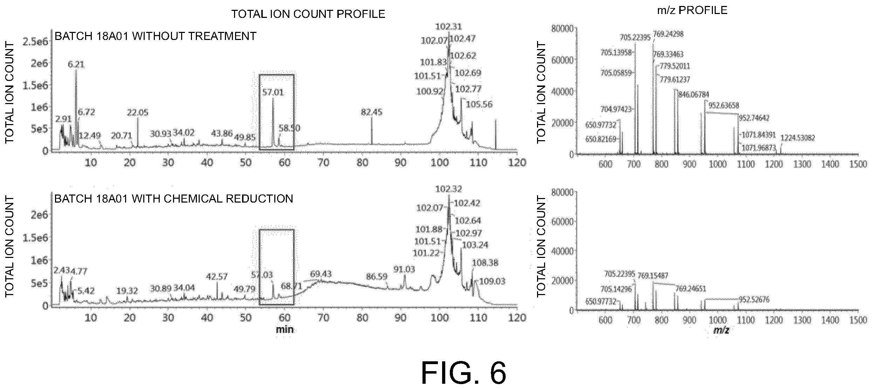

A sample of the dialyzable leukocytes extract reduced with dithiothreitol (DTT) was analyzed under the same analytical conditions to confirm that the m/z signals were attributable to Ub and Ub(-GG) rather than peptide aggregates. The reduced sample also showed the Ub and Ub(-GG) m/z patterns. This is shown in Figure 6.

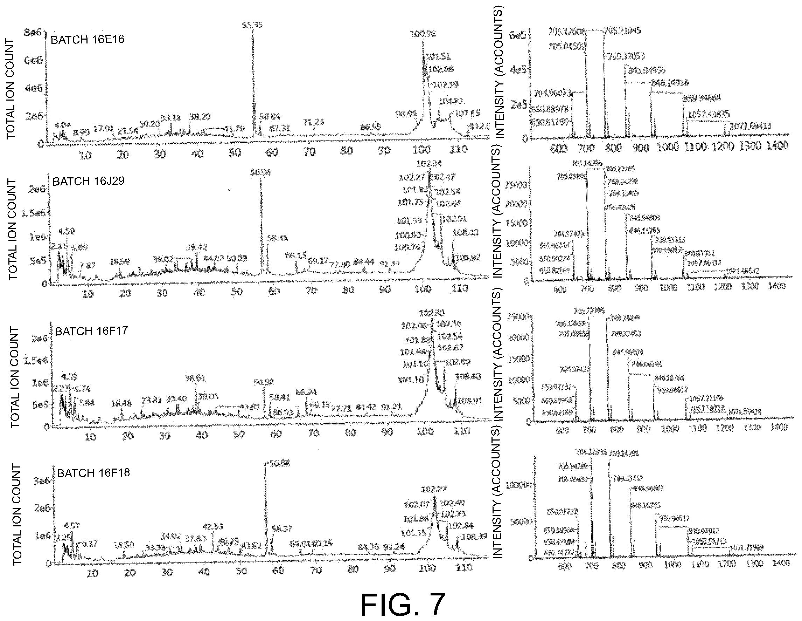

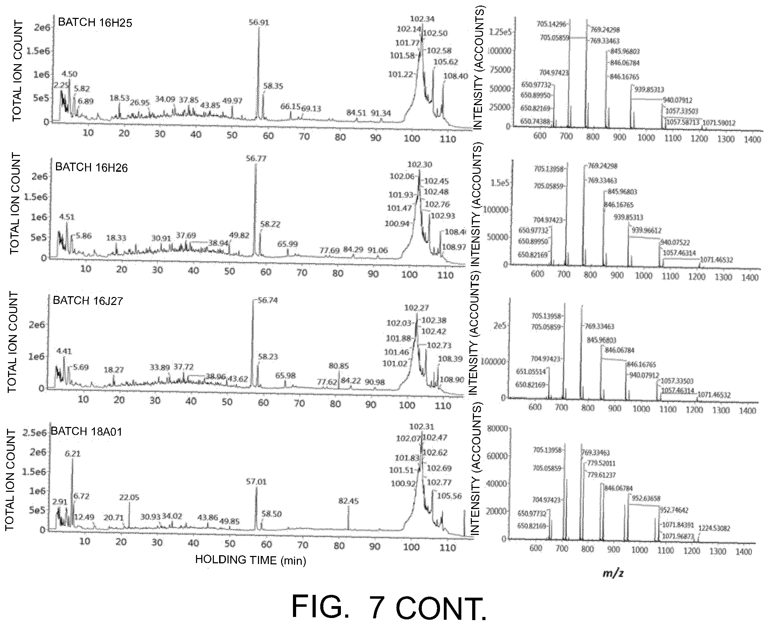

Furthermore, the Ub and Ub(-GG) TIC and m/z profiles (from Figure 6) were reproducible in mass spectrometry analysis of the dialyzable leukocytes extract from 8 batches (in Figure 7).

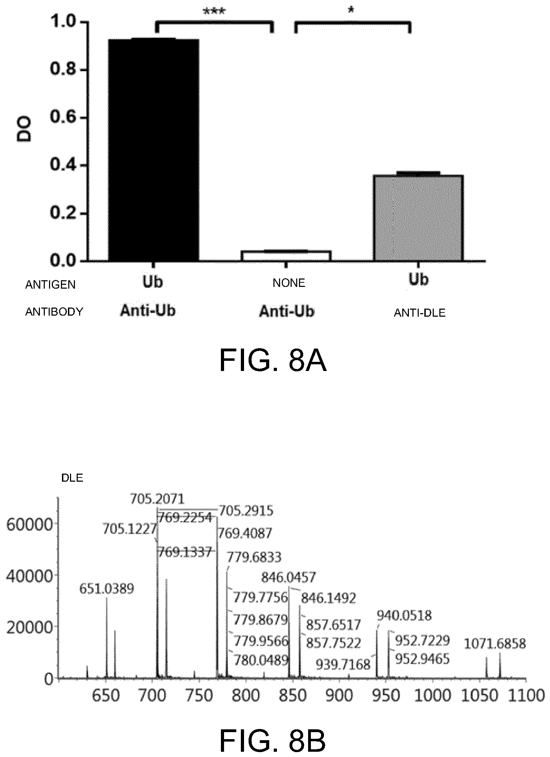

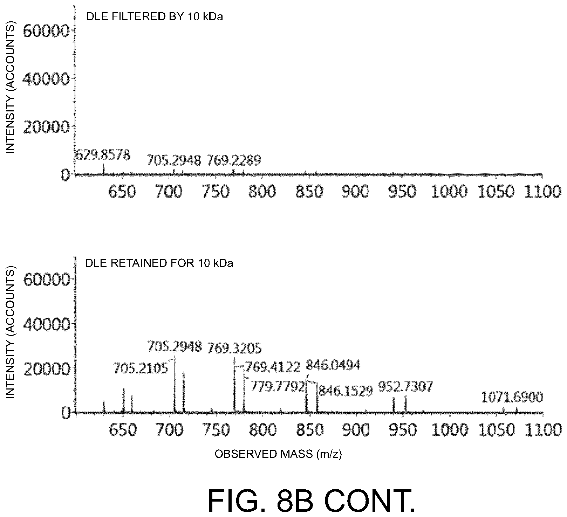

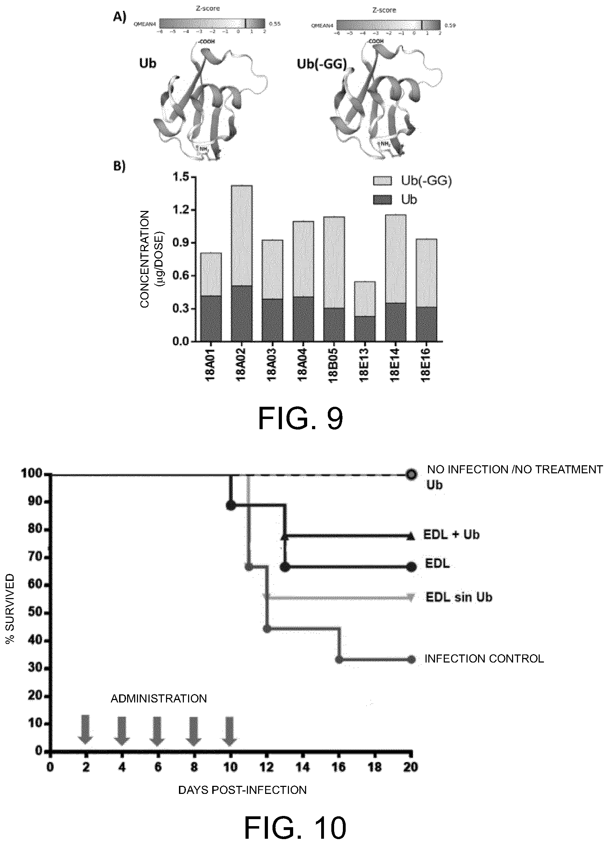

The identity of Ub and Ub(-GG) in the dialyzable leukocytes extract was also determined using polyclonal antibodies. A polyclonal antibody (Mellado-Sanchez, G., et al., Development of Functional Antibodies Directed to Human Dialyzable Leukocyte Extract (Transferon(R)). J Immunol Res, 2019. 2019: p. 2754920.), recognized recombinant human monomeric Ub, as well as a commercial polyclonal anti-Ub antibody (Figure 8A). In addition, a sample of the dialyzable leukocytes extract (2 mg/mL) was incubated with a commercial polyclonal anti-Ub antibody at 4°C overnight and filtered through a 100 kDa membrane. Analysis of the intact mass showed that the anti-Ub antibody retained both Ub and Ub(-GG) during filtration (Figure 8B). This result also confirmed that the two sets of m/z signals co-eluting at 55 min in the TIC profile of the dialyzable leukocytes extract correspond to Ub and Ub(-GG), two structurally and physicochemically similar peptide entities. The structural similarity between Ub and Ub(-GG) was also assessed by homology modeling using the SWISS-MODEL tool. The models obtained for Ub and Ub(-GG) and their Z-score values were similar (0.55 versus 0.59) (Figure 9 in its part a).

Total Ub [Ub + Ub(-GG)] was quantified in eight batches of the dialyzable leukocytes extract (18A01, 18A02, 18A03, 18A04, 18B05, 18E13, 18E14 and 18E16). A 5 mL vial (0.4 mg/mL of the dialyzable leukocytes extract, equivalent to one dose) was lyophilized, reconstituted in water (2 mg/mL) and quantitated by MS using a standard human monomeric Ub curve; results were calculated in µg/dose, which is equivalent to 5 µg/mL. Quantification showed that the mean Ub(-GG) peptide content was almost double that of monomeric Ub (0.637 µg/dose versus 0.366 µg/dose) in the dialyzable leukocytes extract and that the mean total Ub content was 1003 µg/dose (Figure 9 in its part b).

### Example 3. Detection of the complete Ub monomer by means of ELISA.

The presence of monomeric ubiquitin (Ub) in the dialyzable leukocytes extract was indirectly confirmed by indirect ELISA using polyclonal antibodies as in the previous example and the OptEIA^{®} kit according to the manufacturer's instructions. Maxisorp^{®} ELISA plates were coated with 50 µL of recombinant human Ub (10 µg/mL) in carbonate buffer at pH 9.5 (BD Biosciences^{®}) at 4°C overnight. The detection of Ub was achieved using either the polyclonal antibody (1:250 dilution) or a commercial polyclonal anti-Ub antibody (1:300 dilution; Boston Biochem Inc.), both developed in rabbit. Primary antibodies were detected using a horseradish peroxidase-coupled anti-rabbit IgG antibody (1:2500 dilution; Sigma-Aldrich). Data was analyzed at 450 nm/570 nm on an Epoch^{®} spectrophotometer (BioTek^{®}; VT, USA) after carrying out the colorimetric reaction with a 3,3',5,5' tetramethylbenzidine (TMB) (BD Biosciencs). Data was processed using the Gen5 software (BioTek^{®}).

### Example 4. Murine model of HSV-1 infection.

The relevance of the Ub content in a dialyzable leukocytes extract (EDL) was evaluated using a murine model of HSV-1 infection, as previously described (Salinas-Jazmin, N., et al., Herpes murine model as a Biological assay to test dialyzable leukocyte extracts activity. J Immunol Res, 2015. 2015: p. 146305); all procedures were performed blindly by trained investigators. For this, Ub and Ub(-GG) were considered the same molecule (total Ub) considering that they are physiochemically and structurally similar and that the lack of terminal Gly does not affect the biological activity of extracellular Ub.

In the mouse model of HSV-1 infection, BALB/c mice are infected with HSV-1, the drug treatment is administered via the oropharyngeal route at 2, 4, 6, 8, and 10 days after infection, and they remain under observation for ten additional days to assess the percentage of survival. The treatments administered were EDL (0.50 µg/200 µL); the added EDL with monomeric Ub (0.50 µg/200 µL + 0.75 µg/200 µL, respectively); the EDL without Ub (≈ 0.50 µg/200 µL), which was removed with a commercial polyclonal anti-Ub antibody (Figure 8B); and Ub (0.75 µg/200 µL) (Figure 10). In this trial, it was observed that the group treated with EDL+ Ub showed an increased percentage of survival compared to the group treated with EDL alone (77.7% versus 66.6%), while the percentage of survival decreased in the group treated with EDL depleted in Ub compared to the EDL group (55.5% versus 66.6%). Interestingly, the group treated with Ub alone showed 100% survival, as did the negative control (no infection/no treatment) group. The concentration of Ub used for this assay was determined in a previous assay in which concentrations equivalent to less than 50% EDL were found to induce no significant change in survival compared to the infection control (data not shown). Taken together, these results indicate that monomeric Ub is a major active component of the EDL peptide mixture.

The total Ub content of EDL without Ub was removed using a polyclonal anti-Ub antibody (Boston Biochem Inc.) and was used as a negative control in the *in vivo* assay. The blotted samples (lot 19J20) were lyophilized and reconstituted in 2 mg/mL water for injection. A volume of 200 µL was incubated with 2 µL of polyclonal anti-Ub antibody (Boston Biochem Inc.) at 4°C overnight. The sample was then centrifugally filtered through a 50 kDa membrane; the permeate and the retained fractions were analyzed by the intact MS method to confirm the removal of Ub. The permeate was sterilized by filtration using a 0.22 µm membrane (Millipore) and stored at 4°C until use.

In the HSV-I infection model, the administration of Ub (0.087 nM) and peptides with a molecular mass of less than 10 KDa were capable of inducing a protective effect. Surprisingly, when the concentration of Ub in EDL was modified using commercial antibodies against Ub or by adding recombinant monomeric human Ub, the protective capacity of EDL was decreased or increased, respectively, indicating that Ub is one of the active components of EDL.

In accordance with what has been previously described, it can be observed that the peptide composition with immunomodulatory effects has been devised so as not to present variability unlike other leukocyte extracts that vary in their peptide content according to their cellular origin and manufacturing method and for the integral regulation of the immune system, and it will be evident to any skilled person in the art that the embodiments of the composition for immunotherapy as previously described and illustrated in the accompanying drawings, are only illustrative and not limiting of the present invention, since numerous considerable changes in its details are possible without departing from the scope of the invention. For example, the combination of the peptide composition with immunomodulatory effects with other pharmaceutical and/or immunogenic and/or immunostimulatory and/or immunotherapeutic agents is possible, and it can be combined with a physiologically or pharmaceutically acceptable vehicle for its administration.

Therefore, the present invention should not be construed as restricted except as required by the prior art and by the scope of the appended claims.

## Claims

1. A peptide composition with immunomodulatory effects, characterized because it comprises the complete human Ubiquitin (Ub) monomer, and a variant of the human Ubiquitin monomer that lacks two terminal glycines (Ub(-GG)).

2. The peptide composition with immunomodulatory effects according to claim 1, further characterized because the complete Ub monomer has a molecular mass of about 8.56 kDa and the Ub(-GG) monomer variant has a molecular mass of 8.45 kDa.

3. The peptide composition with immunomodulatory effects according to claim 1, further characterized because the complete Ub monomer is selected from the amino acid sequence of SEQ ID NO: 1.

4. The peptide composition with immunomodulatory effects according to claim 1, further characterized because the Ub(-GG) monomer variant is selected from the amino acid sequence of SEQ ID NO: 2.

5. The peptide composition with immunomodulatory effects according to claim 1, further characterized because it comprises from 0.168 to 0.268% (w/w) of the complete Ub monomer and of the Ub(-GG) monomer variant.

6. The peptide composition with immunomodulatory effects according to claim 1, further characterized because it comprises the average content of the Ub(-GG) monomer variant in the peptide composition with immunomodulatory effects in a 2:1 ratio to the average content of the complete Ub monomer.

7. The peptide composition with immunomodulatory effects according to claim 1, further characterized because it additionally comprises at least one peptide fragment of the following human proteins Ankyrin-1, Protein 4.1, Hemoglobin Alpha Subunit, Hemoglobin Beta Subunit, Hemoglobin Delta Subunit, Complement Protein C3, Calpastatin, Alpha-Synuclein, Fibrinogen Alpha Chain, Cytoplasmic Actin 1, Thymosin Beta-4, Thymosin Beta-10, or Zixin.

8. The peptide composition with immunomodulatory effects according to claim 7, further characterized because it comprises from 0.644 to 0.924% (w/w) of at least one Ankyrin-1 fragment; from 0.397 to 0.637% (w/w) of at least one Protein 4.1 fragment; from 0.351 to 0.571% (w/w) of at least one Hemoglobin Alpha Subunit fragment; from 0.367 to 0.547% (w/w) of at least one Hemoglobin Beta Subunit and/or Hemoglobin Delta Subunit fragment; from 0.303 to 0.583% (w/w) of at least one Complement C3 protein fragment; from 0.319 to 0.519% (w/w) of at least one Calpastatin fragment; from 0.198 to 0.298% (w/w) of at least one Alpha-Synuclein fragment; from 0.151 to 0.311% (w/w) of at least one Fibrinogen Alpha Chain fragment; from 0.163 to 0.283% (w/w) of at least one Cytoplasmic Actin 1 fragment; from 0.12 to 0.2% (w/w) of at least one Thymosin Beta-4 and/or Thymosin Beta-10 fragment; and/or from 0.108 to 0.208% (w/w) of at least one Zixin fragment.

9. The peptide composition with immunomodulatory effects according to claim 7, further characterized because each peptide fragment of the human Ankyrin-1 protein corresponds to a fragment of the amino acid sequence of SEQ ID NO: 3 or SEQ ID NO: 4; each peptide fragment of the human protein Protein 4.1 corresponds to a fragment of the amino acid sequence of SEQ ID NO: 5; each peptide fragment of the human Hemoglobin Alpha Subunit protein corresponds to a fragment of the amino acid sequence of SEQ ID NO: 6 or SEQ ID NO: 7; each peptide fragment of the human Hemoglobin Beta Subunit protein corresponds to a fragment of the amino acid sequence of SEQ ID NO:8; each peptide fragment of the human Hemoglobin Delta Subunit protein corresponds to a fragment of the amino acid sequence of SEQ ID NO: 9; each peptide fragment of the human Complement C3 protein corresponds to a fragment of the amino acid sequence of SEQ ID NO: 10; each peptide fragment of the human Calpastatin protein corresponds to a fragment of the amino acid sequence of SEQ ID NO: 11, SEQ ID NO: 12 or SEQ ID NO: 13; each peptide fragment of the human Alpha-Synuclein protein corresponds to a fragment of the amino acid sequence of SEQ ID NO: 14 or SEQ ID NO: 15; each peptide fragment of the human Fibrinogen Alpha Chain protein corresponds to a fragment of the amino acid sequence of SEQ ID NO: 16; each peptide fragment of the human Cytoplasmic Actin 1 protein corresponds to a fragment of the amino acid sequence of SEQ ID NO: 17, SEQ ID NO: 18 or SEQ ID NO: 19; each peptide fragment of the human Thymosin Beta-4 protein corresponds to a fragment of the amino acid sequence of SEQ ID NO: 20; each peptide fragment of the human Thymosin Beta-10 protein corresponds to a fragment of the amino acid sequence of SEQ ID NO: 21; and each peptide fragment of the human Zixin protein corresponds to a fragment of the amino acid sequence of SEQ ID NO: 22.

10. The peptide composition with immunomodulatory effects according to claim 1, further characterized because it is administered in combination with other pharmaceutical and/or immunogenic and/or immunostimulatory and/or immunotherapeutic agents; and a physiologically or pharmaceutically acceptable vehicle.
